# EUROPEAN PATENT APPLICATION

(11) **EP 2 392 310 A1**
(43) Date of publication of application: **07.12.2011**
(21) Application number: 11168842.0
(22) Date of filing: 06.06.2011
(51) Int. Cl.: A61K 6/10, A61C 9/00

(54) **Dental composition**

(30) Priority: 07.06.2010 US 795424
(71) Applicant: Kerr Corporation, Orange, California 92857 (US)
(72) Inventor: Xiangxu, Chen, Diamond Bar, CA California 91765 (US); Xuejun, Quian, Foothill Ranch, CA California 92610 (US)
(74) Representative: Findlay, Alice Rosemary

(57) **Abstract**

A composition is used for temporarily widening a gingival sulcus, the composition comprising a polymerizable monomer having at least one ethylenically unsaturated group, a photo polymerization initiator, a fine inorganic powder, and an astringent. The uncured composition is inserted within a gingival sulcus to be widened, and the composition is thereafter irradiated *in situ* to polymerize the composition. The result is a rubbery material that is easily removable from the widened sulcus.

## Description

The invention relates to gingival retraction for dental and/or medical purposes.

The gingiva is the soft mucosal tissue that connects teeth and bone. It is a common practice for dental practitioners to retract (i.e., temporarily widen) a gingival sulcus for further dental treatments, such as impressions.

Methods to retract a gingival sulcus may be classified as mechanical, chemo-mechanical, rotary curettage, and electro-surgical methods.

Mechanical methods involve placing a string into the gingival sulcus to physically displace the tissue. Gingival retraction cords are commercially available, e.g., Ultrapak^{™} (Ultradent, South Jordan UT). During gingival retraction procedures, gingival reaction cords are packed and maintained between the gingiva and tooth, then are removed before further dental treatments. Dental practitioners generally find retraction cord packing a time-consuming and frustrating procedure. Bleeding and oozing may also result from pressure applied during the procedure.

Chemo-mechanical methods involve treatment with one or more chemicals that may shrink the tissues temporarily and may also control hemorrhage. An astringent agent, also referred to as an astrigent, is such a chemical; it shrinks or constricts body tissues. This effect is usually local after topical application. Astringents have been used in gingival retraction procedures to stop bleeding or oozing. Chemicals commonly used as astringents in the chemo-mechanical method may be alums (sulfates that have the typical formula M⁺₂SO₄·M³⁺₂(SO₄)·24H₂O₃ where M⁺ denotes the sign of an alkali metal or ammonium ion and M³⁺ denotes one of the trivalent metal ions, typically aluminum, chromium, or iron (III)); aluminum chloride; aluminum sulfate; ferric chloride; ferric sulfate; zinc chloride; zinc sulfate; and/or epinephrine. Aluminum chloride, ferric sulfate, and epinephrine are the most widely used astringents. Commercially-available products used in dental clinics include, but are not limited to, ViscoStat® (20% weight percent ferric sulfate, Ultradent Products, South Jordan, UT), ViscoStat® Clear (25% weight percent aluminum chloride, Ultradent Products, South Jordan, UT), Gel-Cord^{™} (25% weight percent aluminum sulfate, Pascal International, Bellevue, WA), an Expasyl® (15% weight percent aluminum chloride, Kerr Corporation, Orange, CA).

Dental tools have been developed to facilitate gingival retraction and may be used alone or with other treatments. For example, lasers can promote gum healing, reattach gum tissues to root surfaces, and destroy bacteria involved in gum diseases.

Such procedures are time-consuming and require skills in application and use, and are exacerbated when gingival retractions are applied on several teeth at the same time. While cordless chemo-mechanical gingival retraction materials have been developed, and while astringent chemicals may be included to effectively cause tissue or blood vessel to contract to further control oozing of gingival tissue, other compositions and methods are desirable.

In one embodiment of the present invention, a composition for use in widening a gingival sulcus is provided. The uncured composition is inserted within a gingival sulcus to be widened. The uncured composition comprises a polymerizable monomer having at least one ethylenically unsaturated group in an amount ranging from about 0.05 weight percent to about 80 weight percent, a photo polymerization initiator in an amount ranging from about 0,001 weight percent to about 5 weight percent, a fine inorganic powder in an amount ranging from about 0.1 weight percent to about 90 weight percent, and an astringent in an amount ranging from about 3weight percent to about 40 weight percent with the proviso that the uncured composition is substantially free of iron (III). The weight percents are based on the total weight of the uncured composition. Moreover, the uncured composition has a viscosity that is higher than about 13,000 Pascals•second and is consistent with penetration into the uncured composition to a range of between about 0.05 mm to about 3 mm, inclusive, using ASTM D-5 with total weight of a plunger and needle of 50 grams, test duration of 10 seconds, and a sample size of 10 mm in diameter and 8 mm in depth. The uncured composition is maintained in the gingival sulcus from about one second to about fifteen minutes before curing, and the uncured composition is then photo cured to provide a cured composition having a cure depth of about 0.5 mm or greater.

According to another embodiment of the present invention, a composition for use in widening a gingival sulcus is provided, the uncured composition being inserted within a gingival sulcus to be widened through a device with a needle. The uncured composition comprises a polymerizable monomer having at least one ethylenically unsaturated group, a photo polymerization initiator, a fine inorganic powder, and an astringent in an amount ranging from about 3weight percent to about 40 weight percent based on the total weight of the composition with the proviso that uncured composition is substantially fee of iron (III). The astringent is an astringent agent selected from the group consisting of alums, aluminum chloride, aluminum sulfate, zinc chloride, zinc sulfate, epinephrine, tannins and combinations thereof. Moreover, the uncured composition has a viscosity that is higher than about 13,000 Pascals•second and is consistent with penetration into the unpolymerized composition to a range of between about 0.05 mm to about 3 mm, inclusive, using ASTM D-5 with total weight of a plunger and needle of 50 grams, test duration of 10 seconds, and a sample size of 10 mm in diameter and 8 mm in depth. The uncured composition is maintained in the gingival sulcus from about one second to about fifteen minutes before curing, and the uncured composition is thereafter photo cured to provide a cured composition having a cure depth of about 0.5 mm or greater, wherein the cured composition is a rubbery material capable of being extended to an extra length of about 0.5% to about 300%.

The invention will now be further described by way of a detailed discussion of preferred embodiments and by way of examples.

There is disclosed a method of widening a gingival sulcus, which is the crevice that surrounds a tooth, is provided using an uncured gingival retraction composition that can be polymerized by light irradiation after gingival placement and can be easily removed after use. In one embodiment, a method for widening a gingival sulcus comprises injecting the uncured composition subsequently described into the gingival sulcus and then polymerizing the composition by light irradiation to form a material that is easily removed with instruments. The cured composition is elastomeric (i.e., rubbery).

Reference throughout this specification to "one embodiment" or "an embodiment" or variation thereof means that a particular feature, structure, material, or characteristic described in connection with the embodiment is included in at least one embodiment, but do not denote that they must be present in every embodiment. Thus, the appearances of the phrases such as "in one embodiment" or "in an embodiment" in various places throughout this specification are not necessarily referring to the same embodiment. Additionally, it is to be understood that "a" or "an" may mean "one or more" unless explicitly staled otherwise. It should be further understood that a weight percent for any given component of the gingival retraction composition is based upon the total weight of the uncured composition.

The uncured gingival retraction composition contains (a) a polymerizable monomer having at least one ethylenically unsaturated group; (b) a photo polymerization initiator; (c) a fine inorganic powder, and (d) an astringent. The uncured composition has a viscosity that is higher than about 13,000 Pascals•second and is consistent with penetration into the unpolymerized composition to a range of between about 0.05 mm to about 3 mm, inclusive, using ASTM D-5 with total weight of a plunger and needle of 50 grams, test duration of 10 seconds, and a sample size of 10 mm in diameter and 8 mm in depth.

Component (a) is a radically polymerizable monomer(s) having one or more ethylenically unsaturated group. The ethylenically unsaturated group may be (meth)acrylate (= acrylate or methacrylate), vinyl, (meth)acrylamide (= acrylamide or methacrylamide) groups. Mono-functional monomers include, but are not limited to, methyl (meth)acrylate, ethyl (meth)acrylate, propyl (meth)acrylate, butyl (meth)acrylate, hydroxyethyl (meth)acrylate, hydroxypropyl (meth)acrylate, glycerol mono(meth)acrylate, polyethyleneglycol mono-(meth)acrylate, polypropyleneglycol mono-(meth)acrylate, polytetramethyleneglycol mono-(meth)acrylate, (meth)acrylamide, N-methyl(meth)acrylamide, N-ethyl(meth)acrylamide, N-butyl(meth)acrylamide, N,N-dimethyl(meth)acrylamide, N,N-diethyl(meth)acrylamide, N,N-dibutyl(meth)acrylamide, (3-acryloylaminopropyl)trimethylammonium chloride, [3-(methacryloylamino)propyl]trimethylammonium chloride, and/or [3-(methacryloylamino)propyl]dimethyl(3-sulfopropyl)ammonium hydroxide. Multifunctional monomers include, but are not limited to, glycerol di(meth)acrylate, glycerol tri(meth)acrylate, 2,2-bis[4-(2-hydroxy-3-methacrloylpropoxy)-phenyl]-propane (bisGMA), urethane di(meth)acrylate, ethoxylated bisphenol A dimethacrylate (EBPADMA-n where n=total number of moles of ethylene oxide in the molecule, as only one example, n = 2-50 units), ethylene glycol di(meth)acrylate, neopentyl glycol di(meth)acrylate, tetraethylene glycol di(meth)acrylate, triethylene glycol di(meth)acrylate, diethylene glycol di(meth)acrylate, 1,3-butylene glycol di(meth)acrylate, cyclohexane dimethanol di(meth)acrylate, 1,6-hexanediol di(meth)acrylate, 1,4-butanediol dimethacrylate, propoxylated glyceryl tri(meth)acrylate, polyethyleneglycol di-(meth)acrylate, polypropyleneglycol di(meth)acrylate, polytetramethyleneglycol di-(meth)acrylate, hexanediol di(meth)acrylate, trimethylolpropane tri(meth)acrylate, ethoxylated trimethylolpropane tri(meth)acrylate, tris (2-hydroxy ethyl) isocyanurate tri(meth)acrylate, pentaerythritol di(meth)acrylate, pentaerythritol tri(meth)acrylate, pentaerythritol tetra(meth)acrylate, dipentaerythritol penta(meth)acrylate, multifunctional aliphatic urethane (meth)acrylate, multifunctional aromatic urethane (meth)acrylate, N,N'-methylenebis-(acrylamide), N,N'-ethylenebis(acrylamide), and/or N-N'-butylenebis(acrylamide).

In one embodiment, the ethylenically unsaturated monomers have flexible units and are rubbery after curing; such a maternal is also referred to as an elastomer. Examples of monomers that can form a rubbery material after curing include, but are not limited to, polyethyleneglycol (PEG) mono- or di- (meth)acrylate with the molecular weight of PEG ranging from 400 to 5000, polypropyleneglycol (PPG) mono- or di- (meth)acrylate with the molecular weight of PPG ranging from 400 to 5000, polyisopropyleneglycol mono- or di-(meth)acrylate with the molecular weight of polyisopropyleneglycol ranging from 400 to 5000, polytetramethyleneglycol mono- or di- (meth)acrylate with the molecular weight of polytetramethyleneglycol ranging from 400 to 5000, EBPADMA-n (where n is greater than 20), ethoxylated trimethylolpropane tri(meth)acrylate) elastomeric urethane (meth)acrylate oligomers that contain PEG or PPG segments with an average molecular weight of 500 to 5000.

In one embodiment, the monomers have a solubility in water of more than 1 wt %. For example, in one embodiment, the monomers have a solubility in water of more than 5 wt %. In one embodiment, the monomers have a solubility in water of more than 15 wt %. Examples of such monomers include, but are not limited to, EBPADMA-n (where n is greater than 20), ethoxylated trimethylolpropane tri(meth)acrylate, polyethylene glycol di-(meth)acrylate, multifunctional aliphatic urethane (meth)acrylate, N,N-diethyl(meth)acrylamide and (3-Acrylamidopropyl)trimethylammonium chloride.

In one embodiment the concentration of ethylenically unsaturated monomer(s) in the total composition from about 0.05 weight percent to about 80 weight percent.

Component (b) is a photo polymerization initiator, also referred to as a photoinitiator, that initiates polymerization of the composition. In one embodiment, a dental curing light capahle of generating ultraviolet (UV) and/or visible light is used. In one embodiment, the concentration of the initiator in the total composition ranges from about 0.001 weight percent to about 5 weight percent.

In one embodiment the photoinitiator is a photosensitizer and a reducing agent. Photo-initiators/sensitizers include, but are not limited to, camphorquinone (CQ), phenathrenequinone, 4,4'-bis(dimethylamino)benzophenone, and/or 4,4'-bis(diethylamino)benzophenone. CQ absorbs both ultraviolet light and visible light Amines, including but not limited to tertiary can be used as reducing agents for CQ to co-initiate free radical polymerization. Examples of tertiary amines include, but are not limited to, ethyl-4-(N,N-dimethylamino) benzoate (EDMAB), 2-ethylhexyl-4-(N,N-dimethylamino) benzoate (ODMAB), 4-dimethylamino-benzophenone (DMABP), p-dimethylamino benzoic acid (DMABA), p-(dimethylamino) benzonitrile (DMABCN), p-(dimethylamino) benzaldehyde, 4'-morpholino-acetophenone, 4'-morpholino-benzophenone, p-dimethylamino) acetophenone, 4,4'-bis(dimethylamino)-benzophenone, 4,4'-bis(diethylamino) benzophenone, and/or dimethylaniline. In one embodiment the tertiary amines EDMAB. ODMAB. DMABP, DMABA and/or DMABCN may be used. Other reducing agents for CQ include, but are not limited to, chemical compounds with urethane and benzhydyl groups.

In one embodiment, the photoinitiator is a phosphine oxide, which includes and multi-acyl phosphine oxide. Phosphine oxides can initiate free radical polymerizations by themselves under UV and/or visible irradiation generated by a typical dental curing device. Examples of phosphine oxides include, but are not limited to, bis(2,4,6-trimethylbenzoyl)-phenyl phosphine oxide (Irgacure 819, Ciba Specialty Chemicals, Basel Switzerland), bis(2,6-dimethoxybenzoyl)-(2,4,4-trimethylpentyl) phosphine oxide (CGI 403, Ciba Specialty Chemicals), and/or ethyl 2,4,6-trimethylbenzoyl-phenyl phosphine oxide (LUCIRIN LR8893X, BASF Corp., Charlotte NC). In one embodiment, two or more phosphine oxides may be combined. An example of combinations of phosphine oxides includes, but is not limited to, a 50:50 by weight mixture of 2,4,6-trimethybenzoyl-diphenyl phosphine oxide and 2-hydroxy-2'methyl-1-phenylpropane-1-one (DAROCUR 4265, Ciba Specialty (Chemicals).

In one embodiment, fluoron and pyronin derivatives initiate free radical polymerization, together with amines and iodonium synergists under UV and/or visible irradiation generated by a typical dental curing device. An example of a fluoron derivative includes, but is not limited to, 5,7-diiodo-3-butoxy-6-fluorone (H-Nu 470, Spectra Group Ltd., Millbury OH). Other examples of fluoron and pyronin derivatives that can initiate free radical polymerizations are described in U.S. Patent Nos. 5,623,080 to Neckers and Shi, and 5,451,343 to Neckers and Shi.

Component (c) is a fine inorganic powder that is incorporated into the composition. A fine powder is one in which the mean particle size is less than about 50 microns. In one embodiment, the inorganic powder is a thickening agent that significantly increases the viscosity of the composition. Examples of fine inorganic powders include, but are not limited to, silicas and clays. Both micrometer size and nanometer size powders can be used in compositions. In one embodiment, the mean particle size is less than about 20 microns. In another embodiment, the mean particle size is less than about 10 microns.

Examples of silicas include, but are not limited to, fumed silica, colloidal silica, and/or precipitated silica. Examples of colloidal and fumed silicas include, but are not limited to, AEROSIL series and AERODISP series (both from Degussa, Ridgefield Park NJ), and CAB-O-SIL series (Cabot Corp., Tuscola IL). AEROSIL series include, but are not limited to, AEROSIL 150, 200, 300, 380, R205, R972, OX-50, OX-130 and OX200 silica. AERODISP series include, but are not limited to, AERODISP W1714, W1824, W1836, W630, W7512S and W7520, all of which are water-based dispersions. CAB-O-SIL series include, but are not limited to, CAB-O-SIL M5, LM-150, TS-720, TS-610, and TS-530. The thickening agent also includes nanoparticles such as those obtained through a solgel process. Examples include those disclosed in U.S. Patent Nos. 4,567,030 to Yuasa et al. and 5,609,675 to Noritake and Yuasa, each of which is expressly incorporated by reference herein. The surface of a silica may be treated or coaled with a coupling agent, such as gamma-methacryloyloxypropyltrimethoxy-silane (MPTMS). In one embodiment, silica has an average particles size of less than 1 micrometer. In another embodiment, silica has an average particle size of 100 nanometers.

Clays are naturally occurring fine-grain particles in sediment, soil, or rock. Clays contain a variety of phyllosilicate minerals rich in silicon, aluminum oxides, hydroxides, and a variety of structural water. Clays are distinguished from other small particles present in sediment/soil/rock, such as silt and sand, by their small size, flake or layered shape, affinity for water, and high plasticity. Clays may have high plasticity when mixed with certain amounts of water. Clays include the following groups: kaolinite, smectite, illite, and chlorite. Kaolinites include the minerals kaolinite, dickite, halloysite, and ancrite. Smectites include pyrophyllite, talc, vermiculite, sauconite, saponite, nontronite, and montmorillonite. Illites include micas. Chlorites include a variety of similar minerals with considerable chemical variation. Clays of kaolinite and smectite groups are used for skin care applications. Montmorillonite is a very soft mineral of the smectite group. It has two tetrahedral sheets sandwiching a central octahedral sheet, also known as a 2:1 clay. Kaolinite has one tetrahedral sheet linked through oxygen atoms to one octahedral sheet of alumina octahedral, also known as a 1:1 clay. Bentonite is a clay consisting mostly of montmorillonite. Bentonite and montmorillonite are sometimes used interchangably to refer to the same mineral. Two types of bentonites exist: sodium bentonite (swelling bentonite) and calcium bentonite (non-swelling bentonite). Bentonites are formed from hydrothermal weathering of volcanic ash. The clay can be a sheet clay, which includes kaolinite, montmorillonite (bentonite), talc, mica (illite), serpentine, chlorite, mullite, kyanite, pumice, goethite, and/or pyrophyllite. In one embodiment, the clay is kaolinite and/or bentonite. In one embodiment, the clay is micronized kaolinite and bentonite.

Examples of other inorganic powders include, but are not limited to, fine particles of metals, metal oxides, metal fluorides, silicates, and aluminosilicates.

In one the concentration of inorganic powder in the total composition ranges between about 0.1 weight percent to about 90 weight percent. In another embodiment, mixtures of different inorganic powders can be used.

Component (d) is an astringent. The astringent may include, but is not limited to, astringent agents, such as aluminum salts, that are commonly used in dental clinics. Exemplary aluminum salts include aluminum chloride, aluminum sulfate, potassium alum, and sodium alum. Other exemplary astringents include zinc chloride, zinc sulfate, tannins, such as gallic acid and flavones, epinephrine and witch hazel.

Unexpectedly, it has been observed that iron (III) salts, such as ferric chloride and ferric sulfate, which are commonly used in dental clinics, interfere with the photo polymerization of the retraction compositions of the present invention. It was discovered that when iron (III) is present in more than an insubstantial amount, the uncured gingival retraction composition does not undergo curing to a depth of at least 0.5 mm, neither under photo polymerization conditions using a photo polymerization initiator nor under thermal radical generation conditions using an azo radical initiator. In view thereof, the uncured gingival retraction compositions should be substantially free of iron (III). In one embodiment, "substantially free of iron (III)" should be understood to denote that the presence of iron (III) is limited to a quantity that will not inhibit the photo polymerization of the uncured composition to a cure depth of at least 0.5 mm after exposure to dental halogen curing light (*e*.*g*., Optilux® 501, 500 mW/cm³) for about 10 seconds.

In another embodiment. "substantially free of iron (III)" means that iron (III) is below detectable limits using standard techniques, such as ASTM D1068, Test Method A, Direct Atomic Absorption. In yet another embodiment, "manufactured free of iron (III)" means that iron (III) is not intentionally added and may merely be present as an impurity of other additives or components of the uncured composition.

In one embodiment, the total concentration of astringent in the gingival retraction composition ranges from about 0.1 weight percent to about 40 weight percent. For example, the total concentration of astringent in the composition may range from about 10 weight percent to about 20 weight percent. In another embodiment, the astringent may be present in an amount ranging from about 20 weight percent to about 40 weight percent.

In one embodiment, the uncured composition may further contain a solvent. The solvent dissolves or disperses monomers, initiators, and other ingredients. The solvent also wets fillers. Both protic and aprotic solvents may be used. A protic solvent is any solvent that carries hydrogen attached to oxygen or nitrogen. Examples of hydrogen attached to oxygen include, but are not limited to, hydroxyl, carboxylic acid, and phosphoric acid groups. An examples of hydrogen attached to nitrogen includes, but is not limited to, an amine group. Protic solvents include, but are not limited to, water, ethyl alcohol, propyl alcohol, isopropyl alcohol, butyl alcohol, *tert*-butyl alcohol, glycerin, polyethylene glycol, polypropylene glycol, pentaerythritol ethoxylate, acetic acid, and/or fatty acids. In one embodiment the concentration of protic solvent(s) in the composition ranges from about 0.1 weight percent to about 60 weight percent. An aprotic solvent is any solvent that does not carry hydrogen attached to oxygen or nitrogen. Aprotic solvents include, but are not limited to, acetone, methyl ethyl ketone, ethyl acetate, tetrahydrofuran, and diethyl ether. In one embodiment, the concentration of aprotic solvent(s) in the composition is in the range of about 0.1 weight percent to about 50 weight percent. In another embodiment, more than one solvent may be used.

In one embodiment, a pH buffering agent may be included to make the composition less acidic and hence more biocompatible. Buffering agents include, but are not limited to, sodium bicarbonate, sodium carbonate, potassium bicarbonate, and/or potassium carbonate, In one embodiment, the total concentration of pH buffering agent in the composition ranges between about 0.01 weight percent to about 10 weight percent. In another embodiment, the total concentration of pH buffering agent in the composition ranges between about 0.1 weight percent to about 5 weight percent.

In one embodiment, a flavorant and/or odorant may be included to impart a more desirable taste and/or smell to the composition. These include, but are not limited to, citrus (e.g., orange, lime), mint (e.g., peppermint), isoamyl acetate, ethyl propionate, and/or ethyl maltol. In one embodiment where a flavorant is included, the concentration of flavorant ranges between about 0.0001 weight percent to about 5 weight percent. In another embodiment where a flavorant is included, the concentration of flavorant ranges between about 0.001 weight percent to about 2 weight percent.

In one embodiment, a colorant may be included to introduce a particular and/or distinctive color to the composition. Colorants include, but are not limited to, dyes, pigments, and inks. In one embodiment, food dyes are used which include, but are not limited to, Brilliant Blue FCF, indigotine, Fast Green FCF, Allura Red AC, tartrazine, and/or Orange Yellow S. In one embodiment where a colorant is used, the concentration of colorant in the composition is between about 0.0001 weight percent to about 3 weight percent. In another embodiment where a colorant is used, the concentration of colorant is between about 0,001 weight percent to about 1 weight percent.

A radiopaque agent with enhanced x-ray absorbing power can be incorporated to increase the radiopacity of the composition. An increased radiopacitiy permits easy detection with X-ray. In one embodiment, the radiopaque agent is an inorganic filler with increased X-ray contract ability. Such inorganic fillers include, but are not limiting to, metals, salts, oxides, fluorides, silicate glass, aluminosilicate glass, aluminoborosilicate glass, and/or fluoroalumininosilicate glass containing elements of high atomic number such as Sr, Y, Zr, Ba, La, Hf, Zn, Bi, W, rare earth metals. Examples include, but not limited to, barium sulfate, silver, strontium fluoride, barium fluoride, ytterbium fluoride, yttrium fluoride, barium tungstate, zinc oxide, bismuth(III) oxide, bariumaluminosilicate, bariumaluminoborosilicate, strontiumaluminosilicate, bariumfluoroaluminosilicate, strontiumfluoroaluminosilicate, strontiumzincfluoroalumino-silicate, and/or zincaluminosilicate. In one embodiment, the fine inorganic powder in the composition functions as a thickening agent as well as a radiopaque agent. In another embodiment, the radiopaque agent is added in addition to the thickening agent.

In one embodiment, a stabilizer is added to obtain a chemically stable composition that has a desirable shelf-life. Stabilizers include, but are not limited to, 3,5-ditert-butyl-4-hydroxytoluene (BHT) and hydroquinone monomethyl ether (MEHQ). In one embodiment, the concentration of the stabilizer is between about 0.0001 weight percent to about 5 weight percent.

The viscosity of the uncured composition is higher than about 13,000 Pascals·second and may be measured by a dynamic stress rheometer. Moreover, the viscosity of the composition may also be measured using, a universal penetrometer according to ASTM D-5, or by other methods known to one skilled in the art.

According to one embodiment, a universal penetrometer is used to measure viscosities of a wide variety of materials using penetration of weighted needles. A plunger is reteased to penetrate into viscous pastes, and depth of penetration is used to compare viscosities. A Precision 73515 (Houston TX) universal penetrometer is employed to evaluate paste viscosities using American Society for Testing and Materials (ASTM) D-5. The total weight of the plunger rod and the penetrating needle is 50 grams and extra weight may be added to bring the total weight of penetration to between 100 grams and 150 grams, The diameter of the penetrating needle is 1 mm. The duration of penetrations is set to be ten seconds. The sample container has a diameter of 10 mm and a depth of 8 mm. Three penetrations may be applied on each freshly prepared sample at 24° C ± 1°C. Using the penetrometer, the probe without additional weight penetrated about 1.1 mm on EXPASYL®) (Kerr, Orange CA). In one embodiment the penetration depth without additional load on the disclosed composition was between about 0.01 mm to about 7.5 mm. In another embodiment, the penetration depth was between about 0.05 mm to about 3 mm. In yet another embodiment, the penetration depth was between about 0.1 mm to about 2 mm.

The composition may be inserted into the gingival sulcus by various methods that include, but are not limited to, an injection device. In one embodiment, the composition is injected into the gingival sulcus using a device having a needle with a diameter between about 0.2 mm to about 2 mm that contacts gingival tissue. In one embodiment, the composition is injected into the gingival sulcus using a device having a needle with a diameter between about 0.7 mm to about 1.6 mm that contacts gingival tissue. Other diameter needles may be determined by actual applications. In one embodiment, the composition remains in the gingival sulcus for between about one second to about fifteen minutes. For example, the composition may remain in the gingival sulcus for between about ten seconds to about five minutes. Due to the high viscosity of the composition, in one embodiment the gingival sulcus is widened to obtain a retraction effect. Multiple injections may be needed to achieve desired retraction. In one embodiment, bleeding of gingival tissue is controlled by an astringent agent, which may also have hemostatic properties. Further, a separate hemostatic agent may be included in the composition.

After the gingival tissue is effectively widened, the composition is then irradiated by a curing light. Curing lights include, but are not limited to, a halogen light and LED (light-emitting diode) light. For example, an Optilux® 501 dental halogen curing light, calibrated for 500 mW/cm³ output, may be used for photo curing. According to embodiments of the present invention, the range of curing time is between about one second to about 300 seconds. It will be appreciated that curing or irradiation time can be varied depending on the practicality imposed by other factors, such as patient comfort and safety. In one embodiment, the curing lime may be about 30 seconds. In another the curing time may be about 20 seconds. In another embodiment, the curing time may be about 10 seconds.

Polymerisation occurs to form a one-piece composition that can be manually removed from the gingival sulcus. The cured gingival retraction composition is cured to a depth of about 0.5 mm or greater. For example, the cure depth may be about 1 mm or greater, about 2 mm or greater, or about 4 mm or greater. Furthermore, the polymerized or cured gingival retraction composition is a rubbery composition that can be extended to an extra length of about 0.5% to about 300%. In one embodiment, a dental instrument is used to remove the polymerized composition.

Materials used for the following examples are set forth in Table 1 below.

**Table 1: Materials used in examples.**

| **Abbreviation** | **Chemical** | **Source** |
|---|---|---|
| Ebecryl 230 | Urethane methacrylate | Cytec Industries |
| SR-252 | Methacrylate monomer | Sartomer |
| SR9036A | Ethoxylated(30)bisphenyl A dimethacrylate | Sartomer |
| BR7432G | High elongation urethane acrylate | Bomar |
| DBP | Dibutyl phthalate | VWR Scientific |
| BHT | 2,6-di-(tert-butyl)-4-methylphenol | Fisher Scientific |
| CQ | Camphorquinone | Hampford Research |
| EDMAB | Ethyl-4-(N,N-dimethylamino) benzoate | Hampford Research |
| OX-50 | Fumed silica | Degussa |
| TS-530 | Surface-treated fumed silica | Cabot Corp. |
| Benlonite | Clay | Southern Clay Products |
| Blue-2 | Indigotine | Warner-Jenkinson |
| Red-40 | Allura Red AC | Spectrum Lab Products |

In the examples below, the uncured compositions were prepared in accordance with the amounts listed. After thorough mixing, the curing property of the sample was evaluated to determine the depth of cure in accordance with Section 7.10 of ANSI/ADA Spec. 27, July 2005. An Optilux® 501 dental halogen curing light, calibrated for 500 mW/cm³ output, was used for photo curing.

### Example 1

A paste was prepared that comprises 9.33 grams of Ebecryl 230, 0.49 gram of DBP, 0.068 gram of CQ, 0.096 gram of EDMAB, and 0.016 gram of BHT. Upon irradiation with a dental halogen curing light for 10 seconds, the depth of cure was 8.6 mm, indicating deep curing.

### Example 2

A paste was prepared that comprises 8.22 grams of Ebecryl 230, 0.43 gram of DBP, 0.06 gram of CQ, 0.085 gram of EDMAB, 0.014 gram of BHT, 9.89 grams of potassium alum. 8.25 grams of OX-50 and 0.002 gram of Blue-2. Upon irradiation with a dental halogen curing light for 10 seconds, the depth of cure was 7.6 mm, indicating deep curing.

### Example 3

A paste was prepared that comprises 9.33 grams of Ebecryl 230. 0.49 gram of DBP, 0,068 gram of CQ. 0.096 gram of EDMAB, 0.016 grain of BHT, 2 grams of iron (III) chloride, and 6 grams of OX-50, Upon irradiation with a dental halogen curing light for 10 seconds, the depth of cure was 0 mm, indicating no noticeable curing.

### Example 4

A paste was prepared that comprises 9.33 grams of Ebecryl 230, 0.49 gram of DBP, 0.068 gram of CQ, 0.096 gram of EDMAB, 0.016 gram of BHT, 0.4 gram of iron (III) chloride, and 10 grams of OX-50. Upon irradiation with a dental halogen curing light for 10 seconds, the depth of cure was 0 mm, indicating no noticeable curing.

### Example 5

A paste was prepared that comprises 8,19 grams of Ebecryl 230. 1.37 grams of DBP, 0.17 gram of CQ, 0.27 gram of EDMAB, 0.014 gram of BHT, 0.4 gram of iron (III) chloride, and 10 grams of OX-50. Upon irradiation with a dental halogen curing light for 10 seconds, the depth of cure was 0 mm, indicating no noticeable curing.

### Example 6

A paste was prepared that comprises 4.6 grams of Ebecryl 230, 0.4 gram of SR252, 0.25 gram of CQ, 0.4 gram of EDMAB, 3 grams of celullose powder, 4 grams of aluminum chloride hexahydrate, 2.6 grams of TS-530, and 0.003 gram Blue-2. Upon irradiation with a dental halogen curing light for 10 seconds, the depth of cure was 3.4 mm, indicating deep curing.

### Example 7

A paste was prepared that comprises 25.36 grams of Ebecryl 230, 2.62 grams of DBP, 0.25 gram of CQ, 0.26 gram of EDMAB, 0.04 grams of BHT, 31.97 grams of aluminum chloride hexahydrate, 34.81 grams of OX-50, and 0.01 gram Blue-2. Upon irradiation with a dental halogen curing light for 10 seconds, the depth of cure was 4.7 mm, indicating deep curing.

### Example 8

A paste was prepared that comprises 25.13 grams of Ebecryl 230, 2.60 gram of DBP, 0.17 gram of CQ, 0.27 gram of EDMAB, 0.04 gram of BHT, 37.8 grams of aluminum sulfate, 30.36 grams of OX-50, and 0.02 gram Blue-2. Upon irradiation with a dental halogen curing light for 10 seconds, the depth of cure was 5.1 mm, indicating deep curing.

### Example 9

A paste was prepared that comprises 20 grams of Ebecryl 230 and 0.6 gram of Phenylbis(2,4,6-trimethylbenzoyl)phosphine oxide. Upon irradiation with a dental halogen curing light for 10 seconds, the depth of cure was more than 2 mm, indicating deep curing.

### Example 10

A paste was prepared that comprises 20 grams of Ebecryl 230, 0.6 gram of Phenylbis(2,4,6-trimethylbenzoyl)phosphine oxide and 5 grams of aluminum chloride hexahydrate. Upon irradiation with a dental halogen curing light for 10 second, the depth of cure was more than 2 mm, indicating deep curing.

### Example 11

A paste was prepared that comprises 20 grams of Ebecryl 230, 0.6 gram of Phenylbis(2,4,6-trimethylbenzoyl)phosphine oxide and 5 grams of iron (III) chloride. Upon irradiation with a dental halogen curing light for 10 seconds, the depth of cure was 0 mm, indicating no noticeable curing.

### Example 12

A paste was prepared that comprises 20 grams of Ebecryl 230, 0.6 gram of Phenylbis(2,4,6-trimethylbenzoyl)phosphine oxide and 1 gram of iron (III) chloride. Upon irradiation with a dental halogen curing light for 10 seconds, the depth of cure was 0 mm, indicating no noticeable curing.

### Example 13

A paste was prepared that comprises 20 grams of Ebecryl 230 and 0.4 gram of 2,2'-Azobis(2-methylpropionitrile). Upon heating to 60° C for 24 hours, the bulk of the paste was fully cured, indicating deep curing.

### Example 14

A paste was prepared that comprises 20 grams of Ebecryl 230, 0.4 gram of 2,2'-Azobis(2-methylpropionitrile) and 5 grams of aluminum chloride hexahydrate. Upon heating to 60° C for 24 hours, the bulk of the paste was fully cured, indicating deep curing.

### Example 15

A paste was prepared that comprises 10 grams of Ebecryl 230, 0.4 gram of 2.2'-Azobis(2-methylpropionitrile) and 5 grams of iron (III) chloride. Upon heating to 60° C for 24 hours, the bulk of the paste has no significant viscosity change, indicating no noticeable curing.

### Example 16

A paste was prepared that comprises 2.0 grams of Ebecryl 230, 0.4 gram of 2,2'-Azobis(2-methylpropionitrile) and 1 gram of iron (III) chloride. Upon heating to 60° C for 24 hours, the bulk of the paste has no significant viscosity change, indicating no noticeable curing.

### Example 17

A paste was prepared that comprises 20 grams of Ebecryl 230, 3 grams of DBP, 0.5 gram of CQ and 0.8 gram of EDMAB. Upon irradiation with a dental halogen curing light for 10 seconds, the depth of cure was 6 mm, indicating deep curing.

### Example 18

A paste was prepared that comprises 20 grams of Ebecryl 230, 3 grams of DBP, 0.5 gram of CQ, 0.8 gram of EDMAB, 0.2 gram of water, and 0.3 gram of Iron (III) chloride. Upon irradiation with a dental halogen curing light for 10 seconds, the depth of cure was 0 mm, indicating no noticeable curing.

### Example 19

A paste was prepared that comprises 20 grams of Ebecryl 230, 3 grams of DBP, 0.5 gram of CQ, 0.8 gram of EDMAB, and 0.8 gram of Red-40. Upon irradiation with a dental halogen curing light for 10 seconds, the depth of cure was 1.4 mm, indicating medium curing.

### Example 20

A paste was prepared that comprises 20 grams of Ebecryl 230, 3 grams of DBP, 0.5 gram of CQ, 0.8 gram of EDMAB, and 2.4 grams of Red-40. Upon irradiation with a dental halogen curing light for 10 seconds, the depth of cure was 0.6 mm, indicating shallow curing.

As shown in Examples 3-5, the presence of iron (III) chloride in a substantial quantity inhibited the photo curing of the compositions. It was unclear whether the red/brownish color of iron (III) chloride was absorbing/blocking the curing light or whether the iron (III) species was with UV-light derived radicals. Without being bound by any particular theory, these two mechanisms were explored. The reduction potential of iron (III), as shown in Examples 13-16, was explored by using an azo radical initiator, which does not require exposure to light. Examples 13 and 14, both of were iron (III) chloride free, demonstrated deep curing upon heating to 60° C for 24 hours, whereas the samples comprising iron (III) chloride (Examples 15 and 16) failed to provide noticeable curing under identical conditions. The UV-light absorption property was explored using an alternative photo polymerisation initiator and a red-colored dye. As shown in Examples 9-12, using a phosphine oxide photo polymerisation initiator in place of camphorquinone still failed to provide adequate curing in the presence of iron (III) chloride. Furthermore, while the presence of a red dye at 3 weight percent (Example 19) and 9 weight percent (Example 20), showed a reduced curing depth from 6 mm (Example 17) to 1.4 mm and 0.6 mm, respectively, it did not completely shut down the photo polymerisation process.

### Examples 21-26

In Examples 21-26, the uncured gingival retraction pastes were prepared according to the relative amounts listed in Table 2 below. The uncured compositions had viscosities greater than 13,000 Pascals·second and were further evaluated using a Precision 73515 (Houston TX) universal penetrometer according to ASTM D-5. Upon irradiation with a dental halogen curing light (Optilux® 501, 500 mW/cm³) for 20 seconds, each of the compositions in Examples 21-26 cured to a rubbery material.

**Table 2. Uncured gingival retraction pastes, reported in weight percent.**

| **EXAMPLE** | **21** | **22** | **23** | **24** | **25** | **26** |
|---|---|---|---|---|---|---|
| SR9036A | 55 | 55 | 44.1 | 34.6 | 37.8 | - |
| BR7432G | - | - | - | - | - | 25.7 |
| CQ | 0.39 | 0.39 | 0.39 | 0.26 | 0.28 | 0.15 |
| EDMAB | 0.55 | 0.55 | 0.44 | 0.35 | 0.38 | 0.37 |
| OX-50 | 22 | 22 | - | - | - | - |
| TS-530 | 22 | 22 | | - | - | - |
| Borosilicate glass | - | - | - | - | - | 34.6 |
| Bentonite | - | - | 44.1 | 51.9 | 47.3 | 14.7 |
| AlCl₃ | - | - | - | - | - | 11 |
| Blue-2 | - | 0.03 | - | | | - |
| Water | - | - | 11 | 13 | 14.2 | 14 |
| Viscosity² | 1.3 | 2.1 | 3.1 | 0.3 | 1.1 | 1.7 |

| | | | | | | |
|---|---|---|---|---|---|---|
| 1. Values listed in Table 2 are weight percents based on the total weight of the uncured composition. 2. Viscosity measured using Precision 73515 penetrometer and reported in millimeters. | | | | | | |

While the present invention has been illustrated by the description of one or more embodiments thereof, and while the embodiments have been described in considerable detail, additional advantages and modifications will readily appear to those skilled in the art.

## Claims

1. A composition for temporarily widening a gingival sulcus, by inserting an uncured composition within a gingival sulcus to be widened, the composition comprising:
a polymerizable monomer having at least one ethylenically unsaturated group in an amount ranging from about 0.05 weight percent to about 80 weight percent,
a photo polymerization initiator in an amount ranging from about 0.001 weight percent to about 5 weight percent,
a fine inorganic powder in an amount ranging from about 0.1 weight percent to about 90 weight percent, and
an astringent in an amount ranging from about 3 weight percent to about 40 weight percent with the proviso that the uncured composition is substantially free of iron (III),
wherein the weight percents are based on the total weight of the uncured composition, and wherein the uncured composition has a viscosity that is higher than about 13,000 Pascals" second and is consistent with penetration into the uncured composition to a range of between about 0.05 mm to about 3 mm, inclusive, using ASTM D-5 with total weight of a plunger and needle of 50 grams, test duration of 10 seconds, and a sample size of 10 mm in diameter and 8 mm in depth;
the uncured composition being maintained in the gingival sulcus from about one second to about fifteen minutes before curing; and the uncured composition then being photo cured to provide a cured composition having a cure depth of about 0.5 mm or greater.

2. The composition of claim 1, wherein the astringent is an astringent agent selected from the group consisting of alums, aluminum chloride, aluminum sulfate, zinc chloride, zinc sulfate, epinephrine, tannins and combinations thereof.

3. The composition of either claim 1 or claim 2, wherein the composition is inserted into the gingival sulcus through a device with a needle.

4. The composition of any preceding claim, wherein the uncured composition further comprises a solvent selected from the group consisting of protic solvents, aprotic solvents, and combinations thereof.

5. The composition of any preceding claim, wherein the ethylenically unsaturated group is selected from the group consisting of acrylate, methacrylate, vinyl, acrylamide, methacrylamide, and combinations thereof.

6. The composition of any preceding claim, wherein the fine inorganic powder has an average particle size of less than about 20 microns.

7. The composition of any preceding claim, wherein the fine inorganic powder has an average particle size of less than 10 microns.

8. The composition of any preceding claim, wherein the fine inorganic powder has an average particle size of less than micron.

9. The composition of any preceding claim, wherein the fine inorganic powder is selected from the group consisting of silica, clay, metal oxide, metal fluoride, silicate, aluminosilicate, and combinations thereof.

10. The composition of any preceding claim, wherein the fine inorganic powder is a radiopaque agent.

11. The composition of any preceding claim, wherein the composition further comprises at least one of a buffering agent a flavorant, an odorant, and/or a colorant.

12. The composition of any preceding claim, wherein the viscosity consistent with penetration into the unpolymerized composition ranges from between about 0.1 mm to about 2 mm, inclusive.

13. The composition of any preceding claim, wherein the photo curing forms a rubbery material capable of being extended to an extra length of about 0.5% to about 300%.

14. The composition of any preceding claim, wherein the uncured composition is manufactured free of iron (III).

15. The composition of any preceding claim, wherein the uncured composition further comprises a dye in an amount less than about 3 weight percent.
